(19) Europäisches Patentamt
European Patent Office
Office européen des brevets

(11) Publication number : 0 375 554 B1

(12) **EUROPEAN PATENT SPECIFICATION**

(45) Date of publication of patent specification : **05.05.93 Bulletin 93/18**

(51) Int. Cl.5 : **C07C 227/32,** C07B 53/00, C07C 229/06

(21) Application number : **89403599.7**

(22) Date of filing : **21.12.89**

(54) **Process for producing optically active threo-dihydroxyphenylserine derivatives.**

(30) Priority : **23.12.88 JP 327144/88**
**20.04.89 JP 102834/89**

(43) Date of publication of application : **27.06.90 Bulletin 90/26**

(45) Publication of the grant of the patent : **05.05.93 Bulletin 93/18**

(84) Designated Contracting States : **CH DE FR GB IT LI**

(56) References cited :
**EP-A- 0 295 890**
**FR-A- 1 589 735**
**TETRAHEDRON LETTERS. vol. 29, no. 48, 1988, OXFORD GB pages 6327 - 6330; T.NISHI ET AL.: "SYNTHESIS OF STATINE AND ITS ANALOGUES BY HOMOGENEOUS ASYMMETRIC HYDROGENATION"**
**JOURNAL OF THE AMERICAN CHEMICAL SOCIETY. vol. 110, no. 2, 06 January 1988, GASTON, PA US pages 629 - 631; M. KITAMURA ET AL.: "HOMOGENEOUS ASYMMETRIC HYDROGENATION OF FUNCTIONALIZED KETONES"**
**JOURNAL OF THE AMERICAN CHEMICAL SOCIETY. vol. 109, no. 19, 16 September 1987, GASTON, PA US pages 5856 - 5858; R. NOYORI ET AL.: "ASYMMETRIC HYDROGENATION OF BETA-KETO CARBOXYLIC ESTERS.A PRACTICAL, PURELY CHEMICAL ACCESS TO BETA-HYDROXY ESTERS IN HIGH ENANTIOMER IC PURITY"**
**JOURNAL OF THE CHEMICAL SOCIETY, CHEMICAL COMMUNICATIONS. no. 13, 1985, LETCHWORTH GB pages 922 - 924; TAKAO IKARIYA ET AL.: "SYNTHESIS OF NOVEL CHIRAL RUTHENIUM COMPLEXES OF 2,2'-BIS(DIPHENYLPHOSPHINO)-1,1'-BINAPHTHYL AND THEIR USE AS ASYMMETRIC CATALYSTS"**

(56) References cited :
**SYNTHETIC COMMUNICATIONS vol. 2, no. 4, 1972, MARCEL DEKKER,INC. pages 237 - 242; M. SUZUKI ET AL: "CONVENIENT SYNTHESES OF AROYLAMINO ACIDS AND ALPHA-AMINO KETONES"**

(73) Proprietor : **SUMITOMO CHEMICAL COMPANY, LIMITED**
**5-33 Kitahama 4-chome Chuo-ku**
**Osaka-shi Osaka (JP)**

(72) Inventor : **Noyori, Ryoji**
**135-417, Nisshincho-Umemorishinden**
**Aichi-gun**
**Aichi 470-01 (JP)**
Inventor : **Kitamura, Masato**
**2-4, Harusatocho Chigusa-ku**
**Nagoya-shi Aichi 464 (JP)**
Inventor : **Ikeda, Takaharu**
**2-1-243, Kuwatacho**
**Ibaraki-shi Osaka 567 (JP)**
Inventor : **Yamachika, Hiroshi**
**3-1-22, Uenonishi**
**Toyonaka-shi Osaka 560 (JP)**
Inventor : **Ishizumi, Kikuo**
**16-15, Uenonishi 2-chome**
**Toyonaka-shi Osaka 560 (JP)**
Inventor : **Terashima, Toru**
**1-2-40, Hiratacho**
**Ibaraki-shi Osaka 567 (JP)**

(74) Representative : **Bourgognon, Jean-Marie et al**
**Cabinet Flechner 22, Avenue de Friedland**
**F-75008 Paris (FR)**

EP 0 375 554 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a process for producing optically active threo-dihydroxyphenylserine derivatives useful as intermediates for L-threo-3-(3,4-dihydroxyphenyl)-serine which has been known to have various pharmacological actions.

Hitherto, L-threo-dihydroxyphenylserine derivatives have been obtained by optical resolution of D,L-threo-dihydroxyphenylserine derivatives. However, according to this process, yield is lower than 50 % and unnecessary D-threo-dihydroxyphenylserine derivatives must be recovered and reused. The recovery and reuse is usually effected by racemization. Two asymmetric carbon atoms in threo-dihydroxyphenylserine derivatives make the racemization hardly proceed in practice, since difficulty is encountered in control of configuration at 2- and 3-positions.

SUMMARY OF THE INVENTION

The object of the present invention is to provide an economically advantageous process for producing optically active threo-dihydroxyphenylserine derivatives without the above-mentioned problems in conventional techniques.

DESCRIPTION OF THE INVENTION

The present invention provides a process for producing optically active threo-dihydroxyphenylserine derivatives represented by the formula (I):

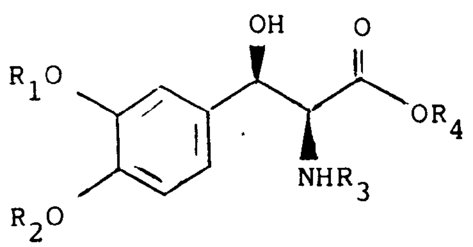

(I)

wherein $R_1$ and $R_2$ each represents or form together a protecting group for the phenolic hydroxyl group, $R_3$ represents a protecting group for the amino group and $R_4$ represents a protecting group for the carboxyl group, which comprises asymmetrically hydrogenating ketones represented by the formula (II):

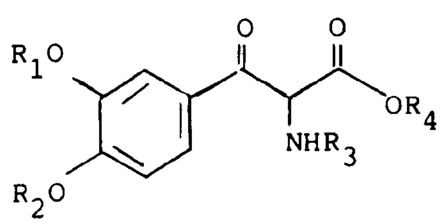

(II)

wherein $R_1$, $R_2$, $R_3$ and $R_4$ are as defined above, in a solvent in the presence of optically active ruthenium phosphine complexes represented by the formula (III):

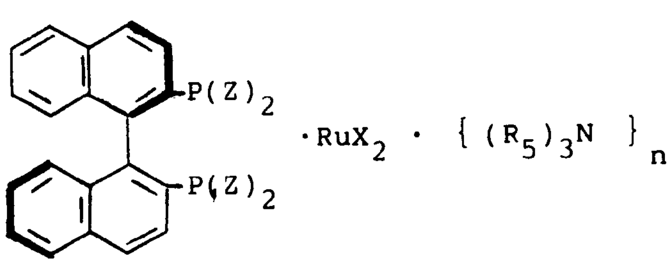

(III)

wherein X represents a chlorine atom, a bromine atom or an acetoxy group, $R_5$ represents a lower alkyl group of 1 - 5 carbon atoms, Z represents a phenyl group or a p-tolyl group and n is 0 or 1.

Any protecting groups for the phenolic hydroxyl group, amino group and carboxyl group in the ketones represented by the formula (II) may be used as long as they are inert for the asymmetric hydrogenation reaction and easily eliminated when the serin derivatives (I) obtained by this reaction are converted to L-threo-3-(3,4-dihydroxyphenyl)-serine.

As the protecting groups for the phenolic hydroxyl group which are represented by $R_1$ and $R_2$ in case of independent protecting groups, mention may be made of, for example, aralkyl groups such as a benzyl group and a p-chlorobenzyl group and lower alkenyl groups such as an allyl group and a 3-butenyl group. The protecting groups which are formed by $R_1$ and $R_2$ together include, for example, a methylene group. The protecting groups for the amino group represented by $R_3$ include, for example, those which form amides such as a formyl group, an acetyl group, a propionyl group and a trifluoroacetyl group and those which form carbamates, e.g., lower alkenyloxycarbonyl groups such as an allyloxycarbonyl group, and a 3-butenyloxycarbonyl group and aralkyloxycarbonyl groups such as a benzyloxycarbonyl group and a p-chlorobenzyloxycarbonyl group. The protecting groups for the carboxyl group which are represented by $R_4$, include, for example, alkyl groups of 1 - 5 carbon atoms, preferably, a methyl group and an ethyl group, aralkyl groups such as a benzyl group and a p-chlorobenzyl group, and lower alkenyl groups such as an allyl group and a 3-butenyl group.

The ketones represented by the formula (II) are produced according to known processes mentioned in, for example, Syn. Commun. 2, 237 (1972). For instance, condensation between ether derivatives of 3,4-dihydroxybenzoyl halides and isocyanoacetates in the presence of a base provides oxazole derivatives which are then hydrolyzed under acidic conditions to obtain salts of 3-(3,4-dihydroxyphenyl)-3-oxo-alanine ester, followed by amidation or carbamation of the amino group of the resulting derivatives until the amino group is protected.

The optically active ruthenium phosphine complexes represented by the formula (III) are produced by known processes, too. For example, $RuBr_2(BINAP)$ is produced by the process mentioned in J.A.C.S. 110 629-631 (1988) and J.C.S. Chem. Commun. 922 (1985). That is, $RuCl_2(COD)$, BINAP and triethylamine are allowed to react to obtain $RuCl_2\cdot(BINAP)\cdot N(C_2H_5)_3$ complex. This complex is allowed to react with sodium acetate to prepare $Ru\cdot(CH_3COO)_2\cdot(BINAP)$, which is then allowed to react with hydrogen bromide to obtain $RuBr_2(BINAP)$. Here, COD denotes cyclo-octa-1,5-diene and BINAP denotes

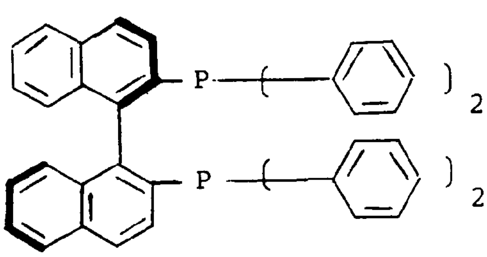

The thus obtained optically active ruthenium phosphine complexes (III) compose optically active phosphine derivatives and ruthenium salts. As the optically active phosphine derivatives, mention may be made of, for example, the above-mentioned BINAP or

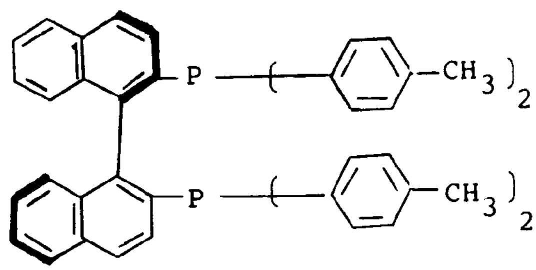

The ruthenium salts include, for example, ruthenium halides such as ruthenium chloride and ruthenium bromide, complex salts of these ruthenium halides with tertiary amines such as trimethylamine, triethylamine, tripropylamine, tributylamine and tripentylamine, and ruthenium acetate. Preferable optically active ruthenium phosphine complexes (III) are, for example, $RuCl_2(BINAP)$, $RuCl_2(BINAP)\cdot N(C_2H_5)_3$, $RuBr_2(BINAP)$ and ruthe-

nium acetate· (BINAP).

The complexes (III) are used in an amount of 0.0001 - 10 mol%, preferably 0.001 - 10 mol% based on the ketone (II).

Hydrogen pressure in the asymmetric hydrogenation reaction of the present invention is 1 kg/$cm^2$ or higher, but preferably 50 - 150 kg/$cm^2$ from a viewpoint of reaction time.

Reaction temperature is 20 - 200 °C and reaction time is normally 20 - 150 hours.

Reaction solvents used in the asymmetric hydrogenation of the present invention may be those which are inert to the reaction. Examples are alcohols such as methanol, ethanol, propyl alcohol and buty alcohol and halogenated hydrocarbons such as chloroform, dichloromethane and 1,2-dichloroethane.

Recovery of complex (III) is usually carried out by adsorption to celite, silica gel and the like or by crystallization. The recovered complex may be reused for the next reaction.

After completion of the reaction, followed by for exmaple, removal of complexes (III), the optically active threo-dihydroxyphenylserine derivatives represented by the formula (I) are obtained by distilling off the reaction solvent. If necessary, purification may be effected by crystallization and the like.

As examples of the thus obtained optically active threo-dihydroxyphenylserine derivatives (I), mention may be made of L-threo-N-acetyl-3-(3,4-methylenedioxyphenyl)serine methyl ester, L-threo-N-benzyloxycarbonyl-3-(3,4-methylenedioxyphenyl)serine methyl ester, L-threo-N-benzyloxycarbonyl-3-(3,4-dibenzyloxyphenyl)-serine benzyl ester, L-threo-N-benzyloxycarbonyl-3-(3,4-dibenzyloxyphenyl)-serine methyl ester, L-threo-N-benzyloxycarbonyl-3-(3,4-diallyloxyphenyl)-serine allyl ester, L-threo-N-allyloxycarbonyl-3-(3,4-diallyloxyphenyl)-serine allyl ester, L-threo-N-propionyl-3-(3,4-methylenedioxyphenyl)serine ethyl ester, L-threo-N-propionyl-3-{3,4-di(3-butenyl)oxyphenyl}-serine ethyl ester, L-threo-N-(3-butenyl)oxycarbonyl-3-{3,4-di(3-butenyl)oxyphenyl}-serine (3-butenyl) ester, and L-threo-N-p-chlorobenzyloxycarbonyl-3-{3,4-di-(p-chlorobenzyl)oxyphenyl}-serine p-chlorobenzyl ester.

The phenylserine derivatives (I) obtained by the present invention are led to L-threo-3-(3,4-dihydroxyphenyl)-serine by elimination of the protecting group. It is preferred that, from a viewpoint of elimination of the protecting group, the protecting group for the phenolic hydroxyl group is the same as that for the carboxyl group and the protecting group for the amino group is one which produces carbamate corresponding to the protecting groups for the phenolic hydroxyl group or the carboxyl group.

According to the present invention, the optically active threo-dihydroxyphenylserine derivatives useful as intermediates for L-threo-3-(3,4-dihydroxyphenyl)-serine are produced from ketones (II), starting racemic compounds, through one step in a high yield. Besides, since the derivatives (I) are of high chemical and optical purity and have high threo isomer ratio, they are easily led to L-threo-3-(3,4-dihydroxyphenyl)-serine by elimination of the protecting groups under proper reaction conditions.

The present invention will be explained by the following examples.

Reference Example 1

(1) Preparation of 3-(3,4-methylenedioxyphenyl)-3-oxo-alanine methyl ester:

Triethylamine (59 ml) and solution of 3,4-methylenedioxybenzoly chloride (23.3 g) in tetrahydrofuran (40 ml) were dropped to solution of methyl $\alpha$-isocyanoacetate (14.2 g) in tetrahydrofuran (250 ml), followed by stirring for 2 days at room temperature. The solvent was distilled off under reduced pressure and the residue was dissolved in ethyl acetate and washed with saturated aqueous sodium chloride solution. The ethyl acetate layer was dried over magnesium sulfate and thereafter the solvent was distilted off under reduced pressure and the residue was recrystallized from methanol to obtain 5-(3,4-methylenedioxy)phenyl-4-carbomethoxy oxazole. m.p. 130 - 132 °C.

This oxazole derivative (2.47 g) was suspended in a mixture of 3N-hydrochloric acid (50 ml) and methanol (100 ml), followed by stirring at 50 - 60 °C for 2 hours. The solvent was distilled off under reduced pressure and the residue was recrystallized from methanolethyl acetate to obtain hydrochloride of 3-(3,4-methylenedioxyphenyl)-3-oxo-alanine methyl ester. m.p. 170 - 171 °C.

(2) Preparation of D,L-N-acetyl-3-(3,4-methylenedioxyphenyl)-3-oxo-alanine methyl ester:

Acetic anhydride (5 ml) was dropped at 20 - 30 °C to the hydrochloride of alanine methyl ester (1 g) which was obtained in (1), followed by stirring at 80 °C for 10 minutes. To the reaction mixture was added water, followed by extraction with ethyl acetate. The organic layer was washed with aqueous sodium bicarbonate solution, followed by removal of aqueous layer. The solvent was distilled off under reduced pressure to obtain D,L-N-acetyl-3-(3,4-methylenedioxyphenyl)-3-oxo-alanine methyl ester. m.p. 99 - 101 °C.

Example 1

D,L-N-acetyl-3-(3,4-methylenedioxyphenyl)-3-oxo-alanine methyl ester (1 g), $RuBr_2$[(R)-BINAP] (12 mg) and dichloromethane (20 ml) were charged in an autoclave in argon gas stream and reaction was allowed to proceed at 50 °C for 120 hours under a hydrogen pressure of 100 $kg/cm^2$ with stirring.

After completion of the reaction, the solvent was distilled off and the concentrated residue was purified by silica gel column chromatography (eluent: chloroform/methanol = 10/1) to obtain L-threo-N-acetyl-3-(3,4-methylenedioxyphenyl)serine methyl ester. (yield: 99.3 %; threo/erythro ratio: 99.6/0.4; L form/D form ratio: 97.1/2.9) m.p.: 182 - 183 °C, $[\alpha]_D^{14}$ +26.4° (c = 0.504, chloroform/methanol = 1/1)

$RuBr_2$[(R)-BINAP] indicates

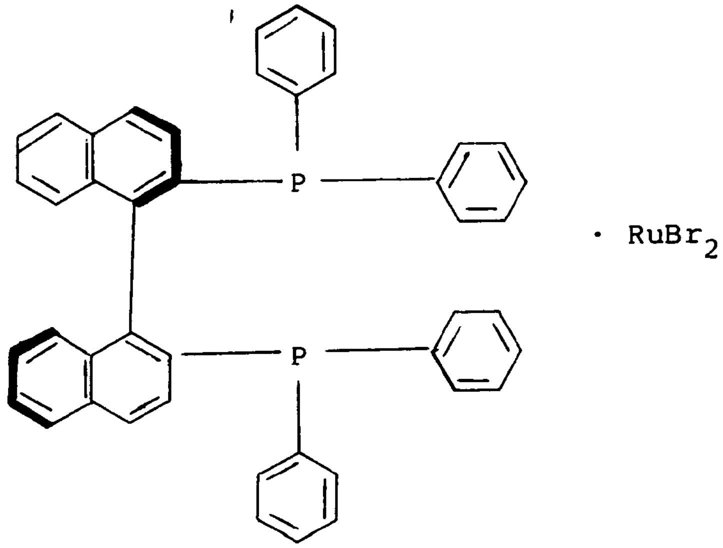

Example 2

Reaction, after-treatment and purification were carried out in the same manner as in Example 1 except that methanol (10 ml) was used in place of the dichloromethane (20 ml) as a solvent for the reaction and reaction time was changed from 120 hours to 36 hours, thereby to obtain L-threo-N-acetyl-3-(3,4-methylenedioxyphenyl)serine methyl ester. (Yield: 100 %; threo/erythro ratio: 92.9/7.1; L form/D form ratio: 93.8/6.2).

Example 3

Reaction, after-treatment and purification were carried out in the same manner as in Example 1 except that $Ru(OAc)_2$[(R)-BINAP] (12 mg) was used as a catalyst in place of the $RuBr_2$[(R)-BINAP], amount of solvent was 5 ml in place of 20 ml and reaction time was 72 hours in place of 120 hours, thereby to obtain L-threo-N-acetyl-3-(3,4-methylenedioxyphenyl)serine methyl ester. (Yield: 85 %; threo/erythro ratio: 98.2/1.8; L form/D form ratio: 96.3/3.7).

$Ru(OAc)_2$[(R)-BINAP] denotes

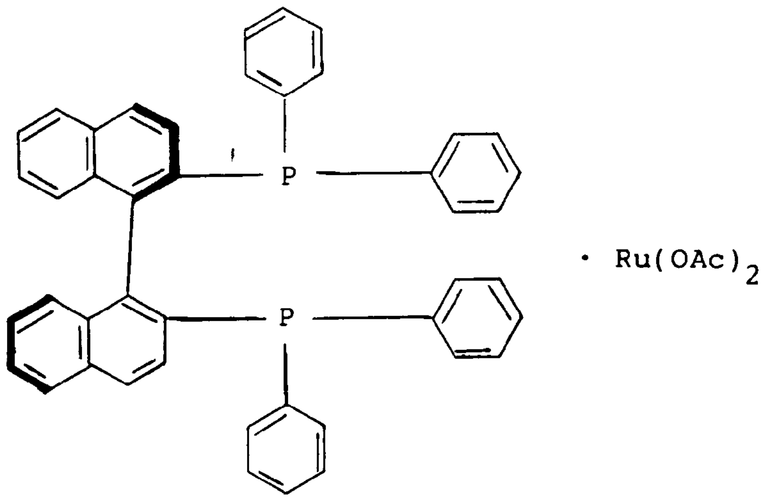

(wherein Ac denotes an acetyl group).

Example 4

Reaction, after-treatment and purification were carried out in the same manner as in Example 1 except that amount of solvent was 5 ml in place of 20 ml and reaction time was 137 hours in place of 120 hours, thereby to obtain L-threo-N-acetyl-3-(3,4-methylenedioxyphenyl)-serine methyl ester. (Yield: 100 %; threo/erythro ratio: 99.1/0.9; L form/D form ratio: 96.1/3.9).

Reference Example 2

Preparation of D,L-N-benzyloxycarbonyl-3-(3,4-methylenedioxyphenyl)-3-oxo-alanine methyl ester:

Reaction, after-treatment and purification were carried out in the same manner as in Reference Example 1 (1) except that benzene was used in place of tetrahydrofuran as a solvent for reaction, thereby to obtain 3-(3,4-methylenedioxyphenyl)-3-oxo-alanine methyl ester hydrochloride.

A solution of KOH (264 mg) in methanol (10 ml) was dropped to a mixture of the above alanine methyl ester hydrochloride (547 mg) and methanol (30 ml) and then benzyl chloroformate (341 mg) was dropped thereto.

After stirring at 5 °C for 2 hours, unsoluble materials were filtered off and the solvent was distilled off under reduced pressure. The residue was purified by silica gel column chromatography (eluent: dichloromethane) and furhter recrystallized from methanol to obtain D,L-N-benzyloxycarbonyl-3-(3,4-methylenedioxyphenyl)-3-oxo-alanine methyl ester. m.p.: 95 - 95.5 °C.

Example 5

Reaction, after-treatment and purification were carried out in the same manner as in Example 1 except that D,L-N-benzyloxycarbonyl-3-(3,4-methylenedioxyphenyl)-3-oxo-alanine methyl ester obtained in Reference Example 2 was used in place of the D,L-N-acetyl-3-(3,4-methylenedioxyphenyl)-3-oxo-alanine methyl ester, thereby to obtain L-threo-N-benzyloxycarbonyl-3-(3,4-methylenedioxyphenyl)-serine methyl ester. (Yield: 97.3 %; threo/erithro ratio: 99.0/1.0; L form/D form ratio: 95.8/4.2). m.p.: 119 - 120 °C, $[\alpha]_D^{14}$ -36.8° (c = 0.544 chloroform).

Example 6

Reaction, after-treatment and purification were carried out in the same manner as in Example 5 except that amount of solvent was 5 ml in place of 20 ml and reaction time was 72 hours in place of 120 hours, thereby to obtain L-threo-N-benzyloxycarbonyl-3-(3,4-methylenedioxyphenyl)-serine methyl ester. (Yield: 100 %; threo/erythro ratio: 98.9/1.1; L form/D form ratio: 96.1/3.9).

Example 7

Reaction, after-treatment and purification were conducted in the same manner as in Example 6 except that reaction temperature was 100 °C in place of 50 °C and reaction time was 96 hours in place of 120 hours, thereby to obtain L-threo-N-benzyloxycarbonyl-3-(3,4-methylenedioxyphenyl)-serine methyl ester. (Yield: 100 %; threo/erythro ratio: 97.2/2.8; L form/D form ratio: 92.7/7.3).

## Claims

1. A process for producing optically active threo-dihydroxyphenylserine derivatives represented by the formula:

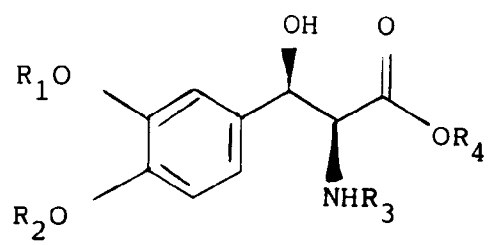

wherein $R_1$ and $R_2$ each represents or form together a protecting group for the phenolic hydroxyl group, $R_3$ represents a protecting group for the amino group and $R_4$ represents a protecting group for the carboxyl group which comprises asymmetrically hydrogenating ketones represented by the formula:

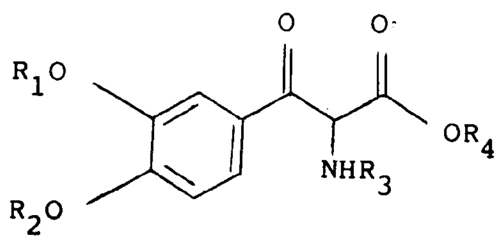

wherein $R_1$, $R_2$, $R_3$ and $R_4$ are as defined above, in a solvent in the presence of optically active ruthenium phosphine complexes represented by the formula:

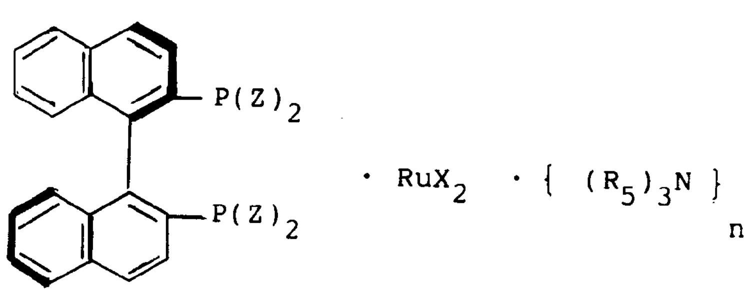

wherein X represents a chlorine atom, a bromine atom or an acetoxy group, $R_5$ represents a lower alkyl group of 1 - 5 carbon atoms, Z represents a phenyl group or a p-tolyl group and n is 0 or 1.

2. A process for producing optically active threo-dihydroxyphenylserine derivatives according to claim 1, wherein $R_1$ and $R_2$ which may be identical or different are a benzyl group, a p-chlorobenzyl group, an allyl group, or a 3-butenyl group or $R_1$ and $R_2$ form together a methylene group.

3. A process for producing optically active threo-dihydroxyphenylserine derivatives according to claim 1,

wherein $R_1$ and $R_2$ form together a methylene group.

4. A process for producing optically active threo-dihydroxyphenylserine derivatives according to any one of claims 1 - 3, wherein $R_3$ is a protecting group which forms an amide.

5. A process for producing optically active threo-dihydroxyphenylserine derivatives according to any one of claims 1 - 3, wherein $R_3$ is a formyl group, an acetyl group, a propionyl group or a trifluoroacetyl group.

6. A process for producing optically active threo-dihydroxyphenylserine derivatives according to any one of claims 1 - 3, wherein $R_3$ is an acetyl group.

7. A process for producing optically active threo-dihydroxyphenylserine derivatives according to any one of claims 1 - 3, wherein $R_3$ is a protecting group which forms a carbamate.

8. A process for producing optically active threo-dihydroxyphenylserine derivatives according to any one of claims 1 - 3, wherein $R_3$ is an allyloxycarbonyl group, a 3-butenyloxycarbonyl group, a benzyloxycarbonyl group or a p-chlorobenzyloxycarbonyl group.

9. A process for producing optically active threo-dihydroxyphenylserine derivatives according to any one of claims 1 - 3, wherein $R_3$ is a benzyloxycarbonyl group.

10. A process for producing optically active threo-dihydroxyphenylserine derivatives according to any one of claims 1 - 9, wherein $R_4$ is a protecting group which forms an ester.

11. A process for producing optically active threo-dihydroxyphenylserine derivatives according to any one of claims 1 - 9, wherein $R_4$ is an alkyl group of 1 - 5 carbon atoms, a benzyl group, a p-chlorobenzyl group, an allyl group, or a 3-butenyl group.

12. A process for producing optically active threo-dihydroxyphenylserine derivatives according to any one of claims 1 - 9, wherein $R_4$ is a methyl group or an ethyl group.

13. A process for producing optically active threo-dihydroxyphenylserine derivatives according to any one of claims 1 - 12, wherein n in the formula of the optically active ruthenium phosphine complexes is 0.

**Patentansprüche**

1. Verfahren zur Herstellung optisch aktiver threo-Dihydroxyphenylserinderivate, dargestellt durch die Formel

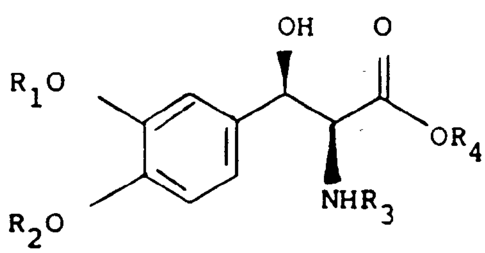

worin $R_1$ und $R_2$ je eine Schutzgruppe für die phenolische Hydroxylgruppe bedeuten oder zusammen bilden, $R_3$ eine Schutzgruppe für die Aminogruppe bedeutet und $R_4$ eine Schutzgruppe für die Carboxylgruppe bedeutet, dadurch **gekennzeichnet**, daß Ketone, dargestellt durch die Formel

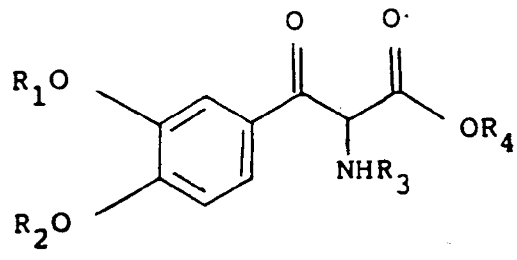

worin $R_1$, $R_2$, $R_3$ und $R_4$ die oben gegebenen Definitionen besitzen, in einem Lösungsmittel in Anwesenheit optisch aktiver Rutheniumphosphinkomplexe, die durch die Formel

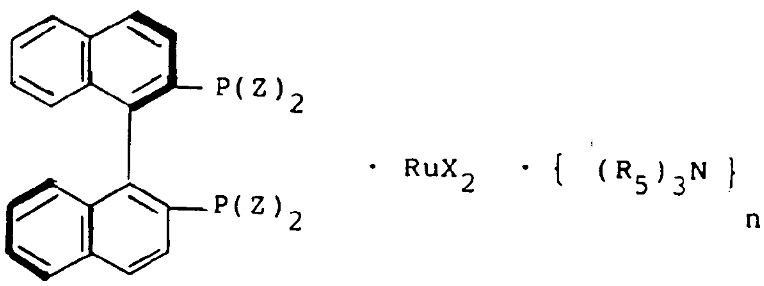

dargestellt werden, worin X ein Chloratom, ein Bromatom oder eine Acetoxygruppe bedeutet, $R_5$ eine Niedrigalkylgruppe mit 1 bis 5 Kohlenstoffatomen bedeutet, Z eine Phenylgruppe oder eine p-Tolylgruppe bedeutet und n 0 oder 1 bedeutet, asymmetrisch hydriert werden.

2. Verfahren zur Herstellung optisch aktiver threo-Dihydroxyphenylserinderivate nach Anspruch 1, dadurch **gekennzeichnet**, daß $R_1$ und $R_2$, die gleich oder unterschiedlich sein können, eine Benzylgruppe, eine p-Chlorbenzylgruppe, eine Allylgruppe oder eine 3-Butenylgruppe bedeuten oder $R_1$ und $R_2$ zusammen eine Methylengruppe bilden.

3. Verfahren zur Herstellung optisch aktiver threo-Dihydroxyphenylserinderivate nach Anspruch 1, dadurch **gekennzeichnet**, daß $R_1$ und $R_2$ zusammen eine Methylengruppe bilden.

4. Verfahren zur Herstellung optisch aktiver threo-Dihydroxyphenylserinderivate nach einem der Ansprüche 1 bis 3, dadurch **gekennzeichnet**, daß $R_3$ eine Schutzgruppe bedeutet, die ein Amid bildet.

5. Verfahren zur Herstellung optisch aktiver threo-Dihydroxyphenylserinderivate nach einem der Ansprüche 1 bis 3, dadurch **gekennzeichnet**, daß $R_3$ eine Formylgruppe, eine Acetylgruppe, eine Propionylgruppe oder eine Trifluoracetylgruppe bedeutet.

6. Verfahren zur Herstellung optisch aktiver threo-Dihydroxyphenylserinderivate nach einem der Ansprüche 1 bis 3, dadurch **gekennzeichnet**, daß $R_3$ eine Acetylgruppe bedeutet.

7. Verfahren zur Herstellung optisch aktiver threo-Dihydroxyphenylserinderivate nach einem der Ansprüche 1 bis 3, dadurch **gekennzeichnet**, daß $R_3$ eine Schutzgruppe bedeutet, die ein Carbamat bildet.

8. Verfahren zur Herstellung optisch aktiver threo-Dihydroxyphenylserinderivate nach einem der Ansprüche 1 bis 3, dadurch **gekennzeichnet**, daß $R_3$ eine Allyloxycarbonylgruppe, eine 3-Butenyloxycarbonylgruppe, eine Benzyloxycarbonylgruppe oder eine p-Chlorbenzyloxycarbonylgruppe bedeutet.

9. Verfahren zur Herstellung optisch aktiver threo-Dihydroxyphenylserinderivate nach einem der Ansprüche 1 bis 3, dadurch **gekennzeichnet**, daß $R_3$ eine Benzyloxycarbonylgruppe bedeutet.

10. Verfahren zur Herstellung optisch aktiver threo-Dihydroxyphenylserinderivate nach einem der Ansprüche 1 bis 9, dadurch **gekennzeichnet**, daß $R_4$ eine Schutzgruppe, die einen Ester bildet, bedeutet.

**11.** Verfahren zur Herstellung optisch aktiver threo-Dihydroxyphenylserinderivate nach einem der Ansprüche 1 bis 9, dadurch **gekennzeichnet**, daß $R_4$ eine Alkylgruppe mit 1 bis 5 Kohlenstoffatomen, eine Benzylgruppe, eine p-Chlorbenzylgruppe, eine Allylgruppe oder eine 3-Butenylgruppe bedeutet.

**12.** Verfahren zur Herstellung optisch aktiver threo-Dihydroxyphenylserinderivate nach einem der Ansprüche 1 bis 9, dadurch **gekennzeichnet**, daß $R_4$ eine Methylgruppe oder eine Ethylgruppe bedeutet.

**13.** Verfahren zur Herstellung optisch aktiver threo-Dihydroxyphenylserinderivate nach einem der Ansprüche 1 bis 12, dadurch **gekennzeichnet**, daß n in der Formel der optisch aktiven Rutheniumphosphinkomplexe 0 bedeutet.

## Revendications

**1.** Procédé de préparation de dérivés de la thréo-dyhydroxyphénylsérine optiquement actifs de formule

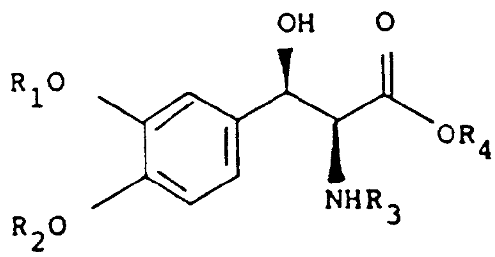

dans laquelle $R_1$ et $R_2$ représentent chacun ou forment ensemble un groupe protecteur du groupe hydroxyle phénolique, $R_3$ représente un groupe protecteur du groupe amino et $R_4$ représente un groupe protecteur du groupe carboxyle, qui consiste à hydrogéner asymétriquement des cétones de formule

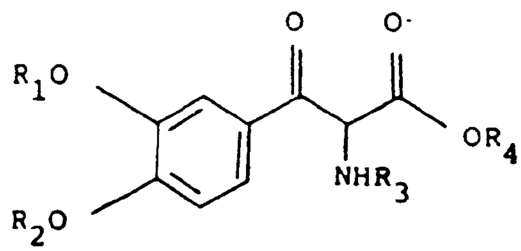

dans laquelle $R_1$, $R_2$, $R_3$ et $R_4$ sont tels que définis ci-dessus, dans un solvant en la présence de complexes ruthénium phosphine optiquement actifs de formule

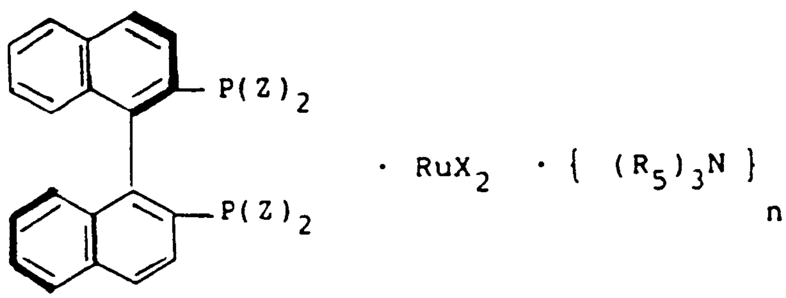

dans laquelle X représente un atome de chlore, un atome de brome ou un groupe acétoxy, $R_5$ représente un groupe alcoyle inférieur de 1 à 5 atomes de carbone, Z représente un groupe phényle ou un groupe p-tolyle et n est 0 ou 1.

2. Procédé de préparation de dérivés de la thréo-dihydroxyphénylsérine optiquement actifs suivant la revendication 1, dans lequel $R_1$ et $R_2$, qui peuvent être identiques ou différents, sont un groupe benzyle, un groupe p-chlorobenzyle, un groupe allyle ou un groupe 3-buténylе ou $R_1$ et $R_2$ forment ensemble un groupe méthylène.

3. Procédé de préparation de dérivés de la thréo-dihydroxyphénylsérine optiquement actifs suivant la revendication 1, dans lequel $R_1$ et $R_2$ forment ensemble un groupe méthyle.

4. Procédé de préparation de dérivés de la thréo-dihydroxyphénylsérine suivant l'une quelconque des revendications 1 à 3, dans lequel $R_3$ est un groupe protecteur qui forme un amide.

5. Procédé de préparation de dérivés de la thréo-dihydroxyphénylsérine suivant l'une quelconque des revendications 1 à 3, dans lequel $R_3$ est un groupe formyle, un groupe acétyle, un groupe propionyle ou un groupe trifluoroacétyle.

6. Procédé de préparation de dérivés de la thréo-dihydroxyphénylsérine suivant l'une quelconque des revendications 1 à 3, dans lequel $R_3$ est un groupe acétyle.

7. Procédé de préparation de dérivés de la thréo-dihydroxyphénylsérine suivant l'une quelconque des revendications 1 à 3, dans lequel $R_3$ est un groupe protecteur qui forme un carbamate.

8. Procédé de préparation de dérivés de la thréo-dihydroxyphénylsérine suivant l'une quelconque des revendications 1 à 3, dans lequel $R_3$ est un groupe allyloxycarbonyle, un groupe 3-buténylоxycarbonyle, un groupe benzyloxycarbonyle ou un groupe p-chlorobenzyloxycarbonyle.

9. Procédé de préparation de dérivés de la thréo-dihydroxyphénylsérine suivant l'une quelconque des revendications 1 à 3, dans lequel $R_3$ est un groupe benzyloxycarbonyle.

10. Procédé de préparation de dérivés de la thréo-dihydroxyphénylsérine suivant l'une quelconque des revendications 1 à 9, dans lequel $R_4$ est un groupe protecteur qui forme un ester.

11. Procédé de préparation de dérivés de la thréo-dihydroxyphénylsérine suivant l'une quelconque des revendications 1 à 9, dans lequel $R_4$ est un groupe alcoyle de 1 à 5 atomes de carbone, un groupe benzyle, un groupe p-chlorobenzyle, un groupe allyle ou un groupe 3-buténylе.

12. Procédé de préparation de dérivés de la thréo-dihydroxyphénylsérine suivant l'une quelconque des revendications 1 à 9, dans lequel $R_4$ est un groupe méthyle ou un groupe éthyle.

13. Procédé de préparation de dérivés de la thréo-dihydroxyphénylsérine suivant l'une quelconque des revendications 1 à 12, dans lequel n dans la formule des complexes ruthénium phosphine optiquement actifs est égal à 0.